# EUROPEAN PATENT SPECIFICATION

(11) **EP 2 331 184 B1**
(45) Date of publication and mention of the grant of the patent: **14.12.2016**
(21) Application number: 09812335.9
(22) Date of filing: 04.09.2009
(51) Int. Cl.: A61M 25/10, A61M 25/06, A61B 17/12, A61B 17/00, A61F 5/00, A61F 2/44, A61B 17/70, A61L 31/04, A61F 2/00, A61F 2/12, A61M 25/00, A61L 31/14, A61B 90/00

(54) **APPARATUS FOR CAPSULE FORMATION IN TISSUE**
VORRICHTUNG ZUR KAPSELBILDUNG IN GEWEBE
APPAREIL POUR LA FORMATION DE CAPSULES DANS UN TISSU

(30) Priority: 05.09.2008 US 191174 P; 04.09.2009 WO PCT/US2009/056123
(43) Date of publication of application: 15.06.2011
(73) Proprietor: Cardiopolymers, Inc, Laguna Hills, CA 92653 (US); Henry Ford Health System, Detroit, MI 48202-3450 (US)
(72) Inventor: AHMANN, Frank, Laguna Hills CA 92653 (US); SABBAH, Hani, N., Waterford MI 48327 (US); SHANNON, Donald, Trabuco Canyon CA 92679 (US)
(74) Representative: Nguyen Van Yen, Christian
(86) International application number: PCT/US2009/056129
(87) International publication number: WO 2010/028310

(56) References cited:
- EP-A1- 0 268 108
- EP-B1- 0 609 386
- WO-A1-01/70114
- WO-A2-2007/075394
- WO-A2-2007/130724
- GB-A- 2 326 344
- US-A- 5 002 556
- US-A- 5 968 004
- US-A1- 2003 004 534
- US-A1- 2005 015 140
- US-A1- 2005 234 498
- US-A1- 2005 245 906
- US-A1- 2006 195 015
- US-A1- 2006 253 126
- US-A1- 2007 073 402
- US-A1- 2008 161 929
- US-B1- 6 420 337

## Description

### BACKGROUND

### Field

The present invention relates to tissue treatment, and more particularly, to apparatus for the emplacement of capsules within tissue.

### SUMMARY

Problems known to those skilled in the art may be overcome by one or more of the embodiments of the invention described herein. For instance, US patent 5 002 556 and European patent application EP0 268 10 disclose apparatuses for inserting inflatable membranes within blood vessels or tissues. But none of these prior art documents disclose a capsule emplacement apparatus capable of first making an opening in tissues and subsequently placing an inflatable membrane within said opening. European patent EP 0 609 386 discloses such devices, but does not disclose an apparatus that contains a filler tube independent from an elongated sealing device, nor a device that allows for optionally sealing an encapsulated membrane. Additional improvements and advantages may be realized by one of ordinary skill in the art upon study of this document. Illustrative embodiments of the invention follow.

According to embodiments, disclosed is a capsule emplacement apparatus comprising: a needle having a tip at a distal end thereof and defining a lumen within; a membrane introducer tube movably disposed within the lumen of the needle, the membrane introducer tube having a distal end; and an encapsulation membrane defining an interior and an inlet region for receiving fluid into the interior through the inlet region, the encapsulation membrane being detatchably coupled to the distal end of the membrane introducer tube near the inlet region, and being controllably expandable upon introduction of the fluid into the interior from a collapsed condition to an expanded condition; and wherein at least one of the needle and the membrane introducer tube is controllably moveable to provide relative motion therebetween for releasing the encapsulation membrane from within the lumen of the needle where the encapsulation membrane is confined in the collapsed condition through the distal
end of the needle, so that the encapsulation membrane is substantially unconfined by the lumen of the needle.

The capsule emplacement apparatus may further comprise an elongated sheath, the needle being movably disposed within a lumen of the sheath. The membrane introducer tube may define a lumen within; and the interior of the encapsulation membrane may be in fluid communication through the inlet region with the lumen of the membrane introducer tube for receiving fluid. The capsule emplacement apparatus may further comprise a filler tube having a distal end and defining a lumen within, wherein: the membrane introducer tube defines a lumen within, the filler tube being movably disposed within the lumen of the membrane introducer tube; and the interior of the encapsulation membrane is in fluid communication through the inlet region with the lumen of the filler tube for receiving fluid.

The capsule emplacement apparatus may further comprise an elongated sealing device; wherein the membrane introducer tube defines a lumen within, the elongated sealing device being movably disposed within the lumen of the membrane introducer tube and positionable proximate the inlet of the encapsulation membrane for sealing the encapsulation membrane. The elongated sealing device may include a welding device. The welding device may include an ultrasonic welder, a radio frequency welder, or a thermal welder.

The elongated sealing device may include an elongated adhesive applicator; and the membrane introducer tube may define a lumen within, the elongated adhesive applicator being movably disposed within the lumen of the membrane introducer tube and positionable proximate the inlet of the encapsulation membrane for sealing the encapsulation membrane. The elongated adhesive applicator may include an applicator tube positionable proximate the inlet of the encapsulation membrane for delivering adhesive into the inlet of the encapsulation membrane.

The capsule emplacement apparatus may further comprise a clamping device for sealing the encapsulation membrane proximate the inlet or a twisting device for sealing the encapsulation membrane proximate the inlet.

According to embodiments, disclosed is a capsule emplacement apparatus, comprising: a source of fluid biomaterial; an elongated sheath having a distal end and a proximal end and defining a lumen within; a needle movably disposed within the lumen of the sheath, the needle having a tip at a distal end thereof and defining a lumen within; a membrane introducer tube movably disposed within the lumen of the needle, the membrane introducer tube having a distal end; an encapsulation membrane defining an interior and an inlet region for receiving fluid into the interior through the inlet region, the encapsulation membrane being detatchably coupled to the distal end of the membrane introducer tube near the inlet region, and being controllably expandable upon introduction of the fluid into the interior from a collapsed condition to an expanded condition; and a control handle disposed at the proximal end of the sheath; the control handle having first controls for advancing the tip of the needle beyond the distal end of the sheath into tissue; the control handle further having second controls for retracting the needle relative to the membrane introducer tube to release the encapsulation membrane from within the lumen of the needle wherein the encapsulation membrane is confined to the collapsed condition, to beyond the distal end of the needle where the encapsulation membrane is unconfined by the lumen of the needle; and the control handle further having third controls for introducing biomaterial from the biomaterial source into the interior of the encapsulation membrane to expand the encapsulation membrane from the collapsed condition to the expanded condition.

The capsule emplacement apparatus may further comprise a filler tube having a distal end and defining a lumen within; wherein the membrane introducer tube defines a lumen within, the filler tube being movably disposed within the lumen of the membrane introducer tube; and wherein the lumen of the filler tube is in fluid communication with the biomaterial source and with the interior of the encapsulation membrane through the inlet region for supplying biomaterial to the interior of the encapsulation membrane.

The membrane introducer tube may define a lumen within, the lumen of the membrane introducer tube being in fluid communication with the biomaterial source and with the interior of the encapsulation membrane through the inlet region for supplying biomaterial to the interior of the encapsulation membrane.

The capsule emplacement apparatus may further comprise an elongated sealing device; wherein the membrane introducer tube defines a lumen within, the elongated sealing device being movably disposed within the lumen of the membrane introducer tube and positionable proximate the inlet of the encapsulation membrane for sealing the encapsulation membrane. The elongated sealing device may include a welding device, an adhesive applicator, a clamping device for sealing the encapsulation membrane proximate the inlet, or a twisting device for sealing the encapsulation membrane proximate the inlet.

### DRAWINGS

The above-mentioned features and objects of the present disclosure will become more apparent with reference to the following description taken in conjunction with the accompanying drawings wherein like reference numerals denote like elements and in which:
Figure 1 illustrates in a broken perspective view an exemplary implementation of an emplacement apparatus;
Figure 2A illustrates in a cut-away view a distal portion of an exemplary implementation of the emplacement apparatus of Figure 1 in a first operational condition;
Figure 2B illustrates in a cut-away view a distal portion of an exemplary implementation of the emplacement apparatus of Figure 1 in a second operational condition;
Figure 2C illustrates in a cut-away view a distal portion of an exemplary implementation of the emplacement apparatus of Figure 1 in a third operational condition;
Figure 2D illustrates in a cut-away view a distal portion of an exemplary implementation of the emplacement apparatus of Figure 1 in a fourth operational condition;
Figure 2E illustrates in a cut-away view a distal portion of an exemplary implementation of the emplacement apparatus of Figure 1 in a fifth operational condition;
Figure 3A illustrates in a cut-away view a distal portion of an exemplary implementation of the emplacement apparatus of Figure 1 in a sixth operational condition;
Figure 3B illustrates in a cut-away view a distal portion of an exemplary implementation of the emplacement apparatus of Figure 1 in a seventh operational condition;
Figure 3C illustrates in a cut-away view a distal portion of an exemplary implementation of the emplacement apparatus of Figure 1 in an eighth operational condition;
Figure 3D illustrates in a cut-away view a distal portion of an exemplary implementation of the emplacement apparatus of Figure 1 in a ninth operational condition;
Figure 4A illustrates in a cut-away view a distal portion of an exemplary implementation of the emplacement apparatus of Figure 1 in an alternative sixth operational condition;
Figure 4B illustrates in a cut-away view a distal portion of an exemplary implementation of the emplacement apparatus of Figure 1 in an alternative seventh operational condition;
Figure 4C illustrates in a cut-away view a distal portion of an exemplary implementation of the emplacement apparatus of Figure 1 in an alternative eighth operational condition;
Figure 4D illustrates in a cut-away view a distal portion of an exemplary implementation of the emplacement apparatus of Figure 1 in an alternative ninth operational condition;
Figure 5 shows a perspective view showing an illustrative implementation of a capsule;
Figure 6 shows a perspective view showing another illustrative implementation of a capsule; and
Figure 7 shows a perspective view showing yet another illustrative implementation of a capsule.

The Figures are to facilitate explanation of the present invention. The number, position, relationship and dimensions of the parts shown in the Figures to form the various implementations described herein, as well as dimensions and dimensional proportions to conform to specific force, weight, strength, flow and similar requirements, are explained herein or are understandable to a person of ordinary skill in the art upon study of this disclosure. Where used in various Figures, the same numerals designate the same or similar parts. Furthermore, when the terms "top," "bottom," "right," "left," "forward," "rear," "first," "second," "inside," "outside," and similar terms are used, the terms should be understood in reference to the orientation of the structures shown in the drawings and utilized to facilitate understanding. Similarly, when the terms "proximal," "distal," and similar positional
terms are used, the terms should be understood in reference to the structures shown in the drawings and utilized to facilitate understanding.

### DETAILED DESCRIPTION

In various treatment regimens, it is advantageous to inject various types of flowable material within tissue of an organ of the body. In the case of injection of a polymer, the polymer may properly cross-link and form a mass within the tissue, but then degrade at such a rate that the therapeutic effect is lost before the therapeutic effect has been entirely realized. The polymer may diffuse within the interstitial spaces of the tissue before cross-linking, which while desirable in some applications, may not be desirable in other applications.

In other instances, one may wish to place a preformed body of material within tissue of an organ of the body. However, emplacement of the body of material within the tissue may require an incision or other such invasive procedure that is traumatic and presents risks to the patient.

Accordingly, there is a need for apparatus to address issues associated with these and other techniques.

Capsules emplacement apparatus are described herein. The emplacement apparatus may be catheter-based or implemented in a handheld unit. The emplacement apparatus serves to introduce an encapsulating membrane in a collapsed condition into the tissue, and then to expand the encapsulating membrane within the tissue into a capsule by injection of fill material into the encapsulating membrane. Other than being capable of injection through the emplacement apparatus, the fill material may be selected and formulated to achieve such mechanical and biological properties within the encapsulating membrane after injection as are desired to achieve the therapeutic effect sought for the patient. Mechanically, the fill material within the encapsulating membrane after injection may be a liquid, a semi-solid such as a gel, or a solid such as a cross-linked polymer. The fill material may or may not be sealed within the encapsulating membrane.

According to embodiments, the emplacement apparatus may be implemented as a catheter having multiple coaxial lumen in the following illustrative manner. The outside of the catheter is an outer guide sheath. A needle tube is operationally movable within the outer guide sheath and is generally coaxial therewith. A membrane introducer tube is operationally movable within the needle tube and generally coaxial therewith. If sealing of the capsule is not desired, fill material may be injected into the encapsulating membrane through the membrane introducer tube. If sealing of the capsule is desired, a filler tube that is generally coaxial with and operationally movable within the membrane introducer tube may be used to fill the encapsulating membrane, and then replaced by a sealing apparatus that is generally coaxial with and operationally movable within the membrane introducer tube. Various sealing apparatus are suitable. Where ultrasonic welding is desired, for example, the sealing apparatus may be an ultrasonic device having a proximal ultrasonic transducer, a transmission member, and a distal sealing tip. Thermal and radio frequency apparatus are also suitable for sealing by welding. Sealing may be done with an adhesive, in which case the sealing apparatus may be an adhesive applicator tube that is generally coaxial with and operationally movable within the membrane introducer tube, and used to apply adhesive to the neck of the filled encapsulating membrane. The filled encapsulating membrane, which is in the form of a capsule, may be released from the membrane introducer tube in any suitable manner.

According to embodiments, the capsule emplacement apparatus is operated as follows. When the needle tube is extended forth from the sheath, the needle is inserted into the tissue with the encapsulation membrane in a collapsed condition and positioned at the end of the membrane introducer tube, inside of and near the tip of the needle. The needle is retracted from the tissue such that the distal, portion of the membrane introducer tube and the encapsulation membrane at the distal end thereof remains within the tissue. Fill material is injected into the encapsulation membrane through a neck region to inflate the encapsulation membrane, for forming a capsule that extends forth from the distal end of the membrane introducer tube. The neck region is sealed using any suitable sealing technique, and the membrane introducer tube is separated from the capsule and removed from the tissue. The capsule remains emplaced within the tissue. The emplacement apparatus may be employed to emplace a plurality of capsules within the tissue at other locations. Capsules may have a variety of different shapes such as, for example, spherical or cylindrical, and different shapes may be placed in different locations.

According to embodiments, the fill material preferably is a biologically compatible natural or synthetic material ("biomaterial") that is flowable under pressure through a lumen and therefore injectable into the body. Once within the encapsulation membrane in situ, the biomaterial may remain in the same flowable state, or may transition from the flowable state to a non-flowable state such as a solid or a semi-solid gel material. The biomaterial may also swell during and/or after the transition. Suitable biomaterial includes a wide variety of substances typically used in medicine and commonly classified as synthetic or natural polymers, copolymers, biopolymers, silicone biomaterials, hydrogels, and composites. Examples of polymers include (a) human or animal-derived such as fibrin glue, collagen, hyaluronic acid, and chitosans; (b) plant-derived polymers such as alginates or biocompatible liquids and gels derived from starch, sugar, and cellulose; (c) lactic acid - based gels and liquids; and (d) synthetically derived materials such as Polyethylene Glycol ("PEG"). The term "biomaterial" may refer to only one biomaterial or a combination of biomaterials, and does not require or preclude the presence of one or more bioreactive components such as (a) cells (stem cells, fibroblasts, skeletal cells, and others); (b) proteins and peptides from the families of growth factors, cytokines, and chemokines; (c) plasmids or genes; (d) natural or engineered binding sites such as RDG binding sites and antibodies and antibody constructs; (e) various pharmaceutical compositions; (f) any other therapeutically beneficial materials; or (g) any combination of the foregoing. Suitable biomaterial also includes silicone and saline.

The encapsulating membrane may be made of any biologically compatible material that is capable of being placed into a collapsed condition and inflatable from the collapsed condition into an inflated condition without damage or rupture to the membrane. Examples of suitable membrane materials include polytetrafluoroethylene ("PTFE"), fluohnated ethylene propylene ("FEP"), low density polyethylene ("LDPE"), polypropylene, polyvinyl chloride ("PVC"), polydimethylsiloxane, polyethylene terephthalate ("PET") (Dacron@), polyamides (nylon), polyether urethane, polycarbonate, polysulfones, polymethyl methacrylate, poly 2-hydroxy-ethylmethachlate (PHEMA), and so forth. The material of the encapsulating membrane may be porous where tissue ingrowth into the encapsulating membrane and/or controlled diffusion of substances from the biomaterial through the encapsulating membrane into the tissue is desired. A suitable porous material is PTFE. On the other hand, the material of the encapsulating membrane may be substantially impermeable where biomaterial is to be securely contained by the encapsulating membrane. A suitable impermeable material is FEP.

The encapsulating membrane may be designed to attain a specific geometric shape when filled with the biomaterial. Depending on the desired therapeutic effect, this shape could be substantially cylindrical, substantially spherical, or any other geometric solid shape.

The fill material and/or the encapsulating membrane may be radiopaque or may contain radiopaque markers to facilitate visualization within the tissue.

Many different techniques are suitable for sealing the capsule, including welding by ultrasound, radio frequency, or thermal energy, bonding by the use of adhesive, and mechanical closure by twisting or clamping. An example of a ultrasound catheter apparatus that may be adapted for the purposes described herein is disclosed in US Patent No. 6,942,677 issued September 13, 2005 to Henry Nita et al.. Where adhesive bonding is desired, a suitable adhesive is a biologically compatible material that flows through one or more lumen for deposition into the neck of the capsule. The adhesive adheres to the encapsulating membrane and forms a seal that seals the neck. In some implementations, the adhesive may be highly viscous and may generally remain highly viscous, while in other exemplary implementations, the adhesive may transition from a flowable to a non-flowable state. The adhesive may be deposited over the capsule neck in the flowable state, and then transition into the non-flowable state to form the seal. Examples of suitable adhesive include medical glues such as the cyanoacrylates and sulfacrylates used in surgery.

According to embodiments, the present application may be applied in a system for volume augmentation of tissue in a living being, such as a human. The system consists generally of an capsule used as a tissue-filling device. The tissue-filling device consists of fill material and an enclosing membrane. According to embodiments, the enclosing membrane forms a container that may be filled.

According to embodiments, the techniques described herein are applicable to many tissues of the body. In cardiac applications, for example, depending on the therapeutic effect sought, a capsule may be emplaced in a single injection site in healthy or diseased tissue of the myocardium, or capsules may be injected into injection sites dispersed over a region of the myocardium or arranged along the myocardium in a therapeutically effective pattern. The techniques described herein are applicable, for example, to the injection of polymer within the ventricular wall of the heart, as described in United States Patent Application Publication No. US 2005/0271631 dated December 8, 2005 (Randall J. Lee et al., Material Compositions and Related Systems and Methods for Treating Cardiac Conditions), and in United States Patent Application Publication No. US 2008/0065046 dated March 13, 2008 (Hani N. Sabbah et al., Intramyocardial Patterning for Global Cardiac Resizing and Reshaping). When so emplaced, the polymer reduces stress within the ventricular wall and, over time, allows the heart to recover functionality. Encapsulation of the polymer within the ventricular wall prevents the polymer from being absorbed by the body before the heart recovers functionality.

Despite continuous improvements in prevention, diagnosis, and treatment of cardiovascular diseases, the prevalence of congestive heart failure (CHF) continues to increase worldwide. With an aging and more sedentary population, CHF is one of the most prevalent chronic diseases in industrialized societies and represents the most common reason for hospitalization in patients who are 65 years and older. In the United States alone, about 5 million Americans are currently affected with the number of patients expected to double by the year 2020 - thus becoming the most common form of heart disease.

The economic impact currently surpasses an estimated $29 billion per year and is expected to rise accordingly. Approximately 60 percent of the cost is taken up by hospitalization-related expenses. Since there is no known cure for CHF (except for a heart transplant), patients are managed with a variety of pharmacologic agents and modification of lifestyle. Ultimately, if patients reach the most advanced stage of heart disease, 50 percent of them die within a year.

CHF occurs when the heart's output or pumping capacity is insufficient for the body's needs. The cause for this deficiency may include several underlying diseases such as ischemia, hypertension, obesity and valvular disease. Irrespective of the underlying cause of CHF, in a large percentage of patients, the left ventricle begins to dilate and enlarge, the muscle wall becomes thinner and the chamber looses its proper shape in an effort to compensate pumping capacity. Once it is set in motion, this negative chain of events results in dilated cardiomyopathy (DCM) and cannot be reversed.

Clinical treatment primarily involves the use of various pharmaceuticals aimed at improving symptoms, reducing the workload of the heart and slowing down the progression of the disease. Stem cells are being used experimentally with marginal benefits to regenerate heart muscle. A number of invasive devices and pump assist devices are being developed with mixed success aimed at slowing the disease progression.

Devices according to embodiments of the present disclosure may prevent or reverse the progression of congestive heart failure (CHF) in patients who have an enlarged left ventricle as a result of mitral valve regurgitation, ischemia, dilated cardiomyopathy and/or other disorders.

Devices according to embodiments of the present disclosure include a biopolymer that is injected using an applicator, directly into strategic areas of the left ventricle muscle during open heart surgery, cather-based procedures, or any other interventional procedure. As it is injected, the biopolymer forms bodies that remain in the heart muscle as permanent implants. These implants provide several beneficial functions: (i) they thicken the muscle wall, (ii) reduce chamber size, (iii) decrease local muscle wall stress, and based on the positioning of the implants, (iv) allow for a re-shaping of the dilated ventricle to impart it a healthier form. Based on the results of studies, these functions lead to a global improvement of the heart as measured by ejection fraction, systolic and diastolic pressures and other biomarkers of stress. The positive effect of the biopolymer implants is measurable within hours of the procedure and remains over the long term.

According to embodiments, the volume augmentation devices described in the present disclosure may be used for tissue bulking in a variety of circumstances, depending on the need. For example: in gastroenterology, wherein increasing the volume of tissue at the gastro-esophageal junction can be used to treat gastro-esophageal reflux disease, and increasing the thickness of the gastric mucosa to decrease the volume of the stomach to treat morbid obesity; in urology, where placing filler radially around the urethra at the neck of the urinary bladder can ameliorate incontinence; and in cardiology, whereby tissue filler may be placed in the ventricular wall to decrease the volume of the left ventricular chamber to treat heart failure, or in the pericardial space to place pressure on the outside of the heart, also intended to decrease the volume of the heart chambers and thereby treat heart failure; and in other applications well known to those skilled in the art. In any of these clinical applications, the tissue-filling device may be combined with any number of other bioactive substances which may be released from the filler itself over time, or be injected concurrently.

According to embodiments, one use of the devices and systems of the present disclosure is in the field of cosmetic plastic surgery wherein the system is used for augmentation in the dermis or subdermis to treat skin contour deficiencies caused by various conditions, including aging, environmental exposure, weight loss, child bearing, surgery, disease such as acne and cancer, or combinations thereof, or for beauty enhancement. The apparatus of the present disclosure is particularly suitable for treating frown lines, worry lines, wrinkles, crow's feet, facial scars, or marionette lines, or to augment facial features such as the lips, cheeks, chin, nose or under the eyes. Treatment of a patient may consist solely of using a tissue-filing device, or the tissue-filling device may be used as part of additional cosmetic surgery such as a face or brow lift. The characteristic of change from first configuration to second configuration makes the tissue-filling device desirable for use in endoscopic surgery. The tissue augmentation device may also be used for breast augmentation, and regions of the body that need volume enlargement during reconstructive plastic surgery, such as after trauma or tumor resection.

The present disclosure addresses those aspects of designing an optimized composition for tissues that need to be repaired. The injectable composition of this disclosure is also suitable for the treatment of many tissue conditions such as augmentation and strengthening of tissue in patients. Other than the plastic surgery or reconstructive surgery, tissue fillers can be used to correct aphonia or dysphonia caused by paralysis of the vocal cords, to correct defect or injury, to the augmentation of hypoplastic breast, to the augmentation of scar tissue, to the treatment of urological disorders (e.g. urinary incontinence), to the treatment of incompetent anal sphincters, to the treatment of vesicoureteral reflux, and to the treatment of gastric fluid reflux by endoscopic or subcutaneous injection of biocompatible hollow particular implants into the submucosal or dermal tissue. Since the disclosure is closely related to the treatment of soft tissue augmentation, it will be described in details by reference hereto.

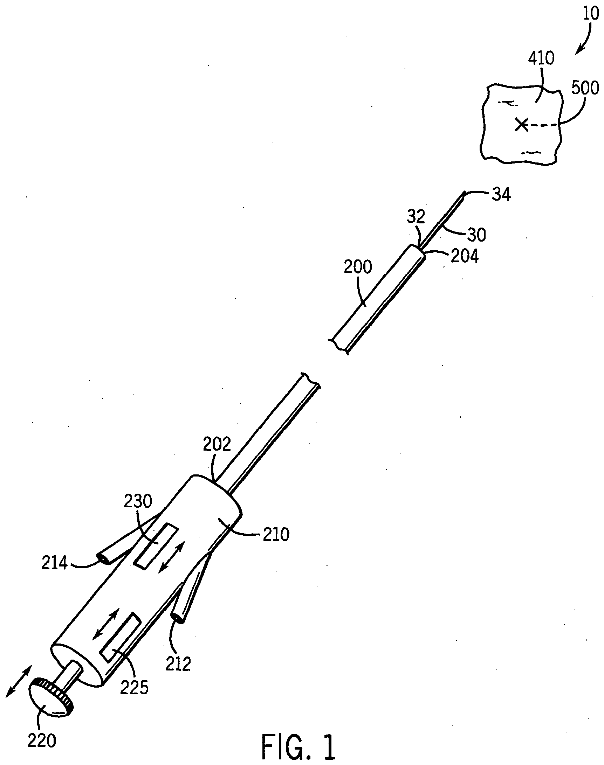

Figure 1 illustrates by broken perspective view an implementation of a capsule emplacement apparatus 10. The emplacement apparatus 10 includes a sheath 200, a handle 210 and a needle 30. The handle 210 is secured to a sheath proximal end 202 of the sheath 200, and the needle 30 is shown as extending forth from the sheath distal end 204 of the sheath 200 generally oriented toward an injection site 500. When so deployed from the sheath distal end 204 of the sheath 200, the needle 30 may be inserted into the tissue 410 at the injection site 500. In other operational conditions, the needle 30 including needle tip 34 may be enclosed within the sheath 200 generally proximate the sheath distal end 204 so that the sheath distal end 204 is atraumatic to allow the sheath distal end 204 to be navigated to a location proximate to the injection site 500.

In some implementations of the emplacement apparatus 10, the sheath 200 may be flexible to allow the sheath distal end 204 of the sheath 200 to be navigated to the injection site 500 through various bodily passages, and the flexibility and other mechanical characteristics of the sheath 200 may vary along the length of the sheath 200. In other implementations, the sheath may be generally rigid, and the sheath distal end may be passed, for example, to the injection site 500 through surgical incision(s) that expose the injection site 500. Radiopaque markings (not shown) may be located about portions of the sheath 200 to assist the physician in the navigation of the sheath distal end 204 of the sheath 200 to the injection site 500 in various implementations.

In the illustrative implementation of the emplacement apparatus 10 shown in Figure 1 , the sheath proximal end 202 is secured to handle 210, which allows the physician to manipulate the sheath 200 in order to direct the sheath distal end 204 to the injection site 500. The handle 210 further includes features that allow the physician to emplace a capsule within tissue at the injection site 500.

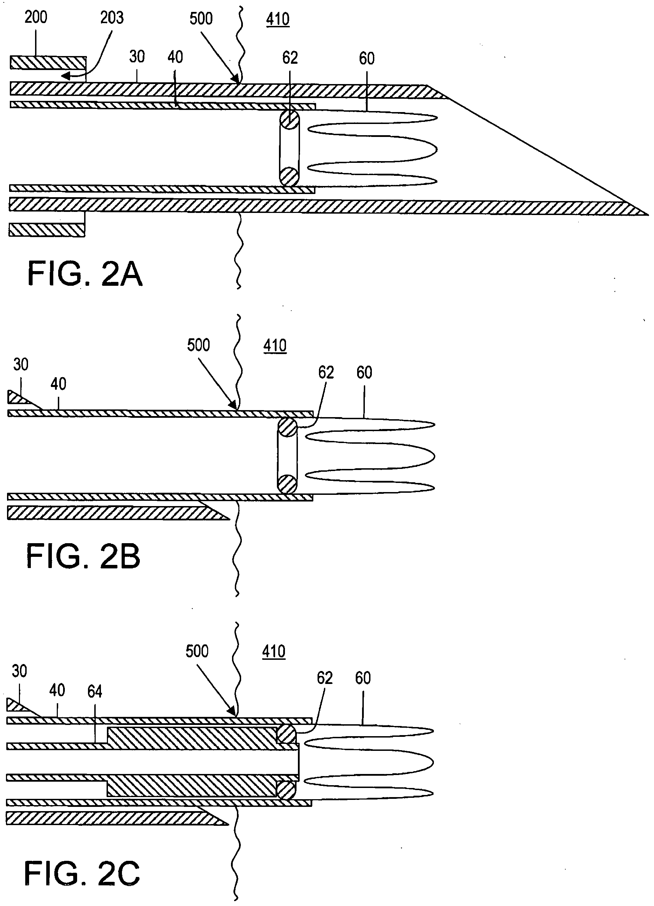

As illustrated, these features include a knob 220 that is slideably secured to the handle 210 so that the knob 220 may be slideably advanced in the distal direction or slideably retracted in the proximal direction by the physician. The knob 220 is in mechanical cooperation with the needle 30 via any suitable mechanism so that, when the knob 220 is advanced in the distal direction, the needle 30 is deployed from the sheath distal end 204 to allow the needle 30 to be inserted into the tissue 410 at the injection site 500. Similarly, when the knob 220 is retracted in the proximal direction, the needle 30 is retracted in the proximal direction and, in this manner, may be retracted from the tissue 410 into the sheath 200. Suitable mechanisms include a needle tube that may be generally coaxial with the sheath, and a deployment assembly mounted in the distal part of the sheath which extends and withdraws the needle under wire control. In this implementation, slides 225, 230 are placed about the handle 210 to cooperate mechanically with, respectively, an encapsulating membrane introducer tube 40 (see, for example, Figures 2A to 2E) and various tubes and devices that are operationally inserted into and moved within the membrane introducer tube 40, such as a fill tube, a sealing apparatus such as a tubular ultrasonic device, and an adhesive applicator tube.

Slides 225 and 230 are advanced or retracted to exert control, and various gears and other mechanical couplings are provided about the handle 210 and/or about the sheath 200 so that the needle 30 cooperates mechanically with the knob 220, the membrane introducer tube 40 cooperates mechanically with slide 225, and the fill tube and ultrasonic device or applicator tube cooperate mechanically with slide 230.

In the implementation illustrated in Figure 1 , ports 212, 214 are located about the handle 210 and/or the sheath 200 generally proximate the sheath proximal end 202. Port 212 communicates with various lumen generally defined within the handle 210 and/or the sheath 200 to communicate biomaterial and adhesive to the appropriate lumen of the emplacement apparatus 10. Alternatively, a reservoir of the biomaterial and/or a reservoir of the adhesive may be located within the emplacement apparatus 10. In various implementations of the emplacement apparatus 10, the sheath 200 and/or the handle 210 may include additional lumen and/or ports through which guide wires may be passed and/or various other fluids may be communicated.

Figures 2A to 2E, 3A to 3D and 4A to 4D show portions of the illustrative emplacement apparatus 10 generally proximate the distal end thereof in various operational conditions.

As illustrated in Figure 2A, the sheath 200 is tubular and defines a sheath lumen 203. The needle 30 and the membrane introducer tube 40 are illustrated as received in part within the sheath lumen 203 and deployed distally so that portions of the needle 30 and the membrane introducer tube 40 extend distal of the distal end of the sheath 200 in this operational condition. In other operational conditions (not shown) the needle 30 and the membrane introducer tube 40 may be positioned entirely within the sheath lumen 203 such that the tip of the needle 30 and the distal end of the membrane introducer tube 40 are contained.

As further illustrated in Figure 2A, the needle 30 defines a needle lumen. Illustratively, the tip of the needle 30 is beveled to form a cutting edge for ease of penetration into tissue 410. The needle 30 is inserted into the tissue 410 at the injection site 500, and the tissue 410 is generally biased about the outer surface of the needle 30.

The membrane introducer tube 40 defines a lumen, and includes at its distal end an encapsulation membrane 60 in a collapsed position. The encapsulation membrane 60 is collapsed into a number of folds and is secured to the inner surface of the membrane introducer tube 40 generally in the area of seal 62. The membrane introducer tube 40 is moveably received within the lumen of the needle 30 such that the distal end of the membrane introducer tube 40 and the tip of the needle 30 are positionable with respect to one another. In the operational condition illustrated in Figure 2A, the distal end of the membrane introducer tube 40 is positioned within the lumen of the needle 30 and near the tip thereof. When the needle 30 penetrates the injection site 500 and is advanced into the tissue 410, the encapsulation membrane 60 is brought into position within the tissue 410.

In the operational condition of the emplacement apparatus 10 shown in Figure 2B, the needle 30 is retracted proximally with respect to the distal end of the membrane introducer tube 40 to withdraw it from the tissue 410. The position of the distal end of the membrane introducer tube 40 is generally maintained while the needle 30 is being retracted from the tissue 410, so that the encapsulation membrane 60 remains in the tissue 410 in a collapsed condition.

In the operational condition of the emplacement apparatus 10 shown in Figure 2C, a fill tube 64 is advanced through the lumen of the membrane introducer tube 40 until it presses up against and engages the seal 62.

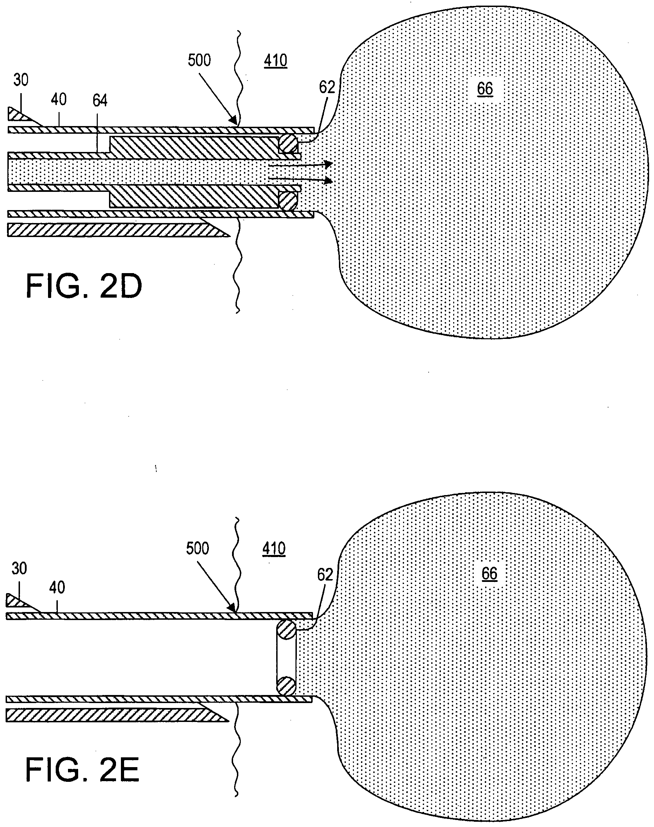

In the operational condition of the emplacement apparatus 10 shown in Figure 2D, biomaterial is injected through the fill tube 64 to expand the encapsulation membrane 60 into capsule 66.

In the operational condition of the emplacement apparatus 10 shown in Figure 2E, the fill tube 64 is withdrawn from the membrane introducer tube 40.

While sealing of the capsule 66 is generally advantageous, it may not be necessary when the biomaterial contained in the capsule 66 becomes sufficient rigid or semi-rigid and other undesirable effects of exposing the biomaterial to body fluids are not troublesome. If the capsule 66 is not to be sealed, the capsule 66 is detached from the membrane introducer tube 40 using any suitable technique, and the membrane introducer tube 40 is withdrawn from the tissue 410 to leave the capsule 66 within the tissue 410. Suitable techniques for detaching the capsule 66 from the membrane introducer tube 40 include twisting the membrane introducer tube 40 to rupture the neck of the capsule 66 or dislodge the seal 62 from the lumen of the membrane introducer tube 40, advancing a plunger through the lumen of the membrane introducer tube 40 and against the seal 62 to push the seal 62 and the neck of the capsule 66 out of the distal end of the membrane introducer tube 40, and forcibly withdrawing the membrane introducer tube 40 from the tissue 410 so that the seal 62 and the neck of the capsule 66 are pulled out of the membrane introducer tube 40 as the capsule 66 is held in place within the tissue 410, which is biased against it. Since various techniques may be used to secure the encapsulation membrane 60 and the seal 62 to the lumen of the membrane introducer tube 40, including press-fit, adhesive, and welding, the choice of securing technique and detachment technique are preferably coordinated.

If the capsule 66 need not be sealed, an alternative technique may be practiced in which the encapsulation membrane 60 is filled directly through the lumen of the membrane introducer tube 40. In this alternative technique, the seal 62 and the fill tube 64 need not be used.

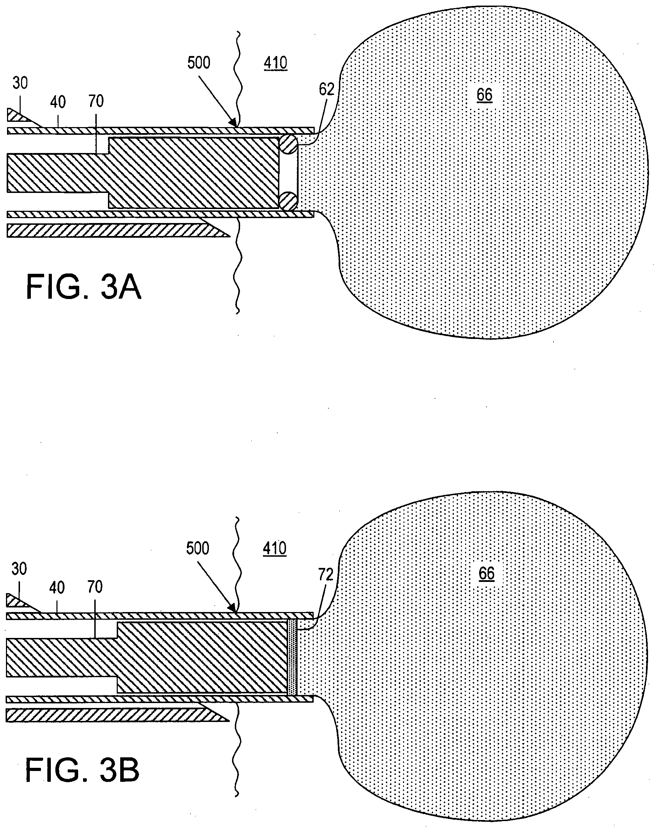

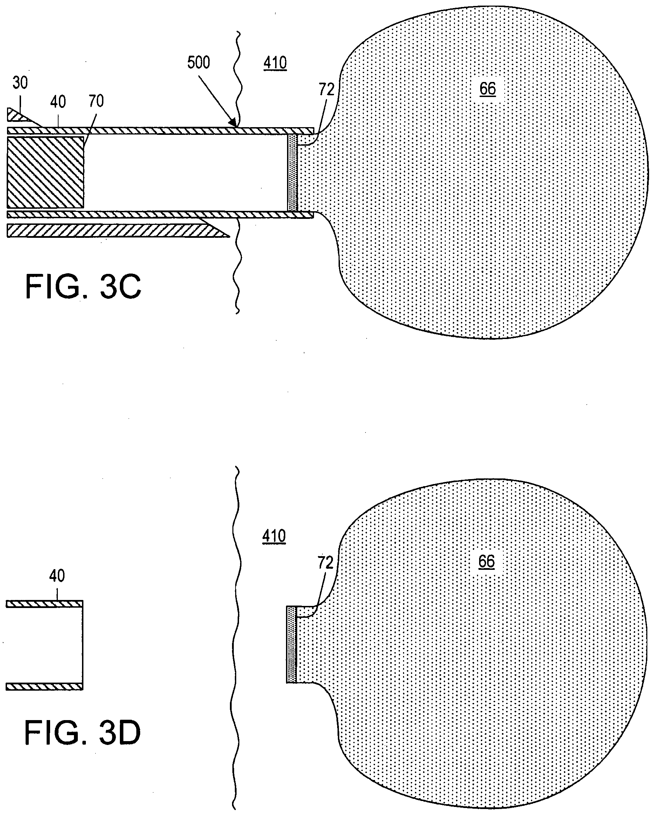

If one desires to seal the capsule 66, an ultrasonic welding technique as operationally shown in Figures 3A to 3D may be used. As shown in Figure 3A, a suitable elongated ultrasonic welding device is advanced through the lumen of the membrane introducer tube 40 until the distal welding tip 70 of the device engages the seal 62 and is slightly biased against the seal 62. The seal 62 is made of a suitable material such as a pliable biologically compatible plastic that softens and flows when subjected to ultrasonic energy, so that upon activation of the ultrasonic welding device as shown in Figure 3B, the seal 62 flows to form plug 72. The welding tip 70 is withdrawn from the plug 72 as shown in Figure 3C, leaving the plug 72 in place in the neck of the capsule 66. When the capsule 66 as sealed by the plug 72 is detached from the membrane introducer tube 40 as described previously, and the membrane introducer tube 40 is withdrawn from the tissue 410 as shown in Figure 3D, the sealed capsule 66 remains in place within the tissue 410.

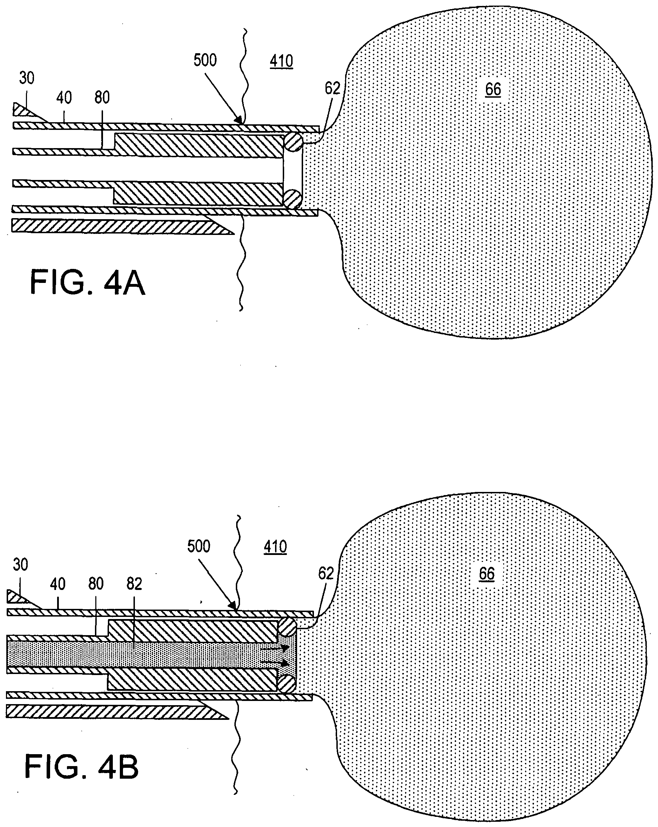

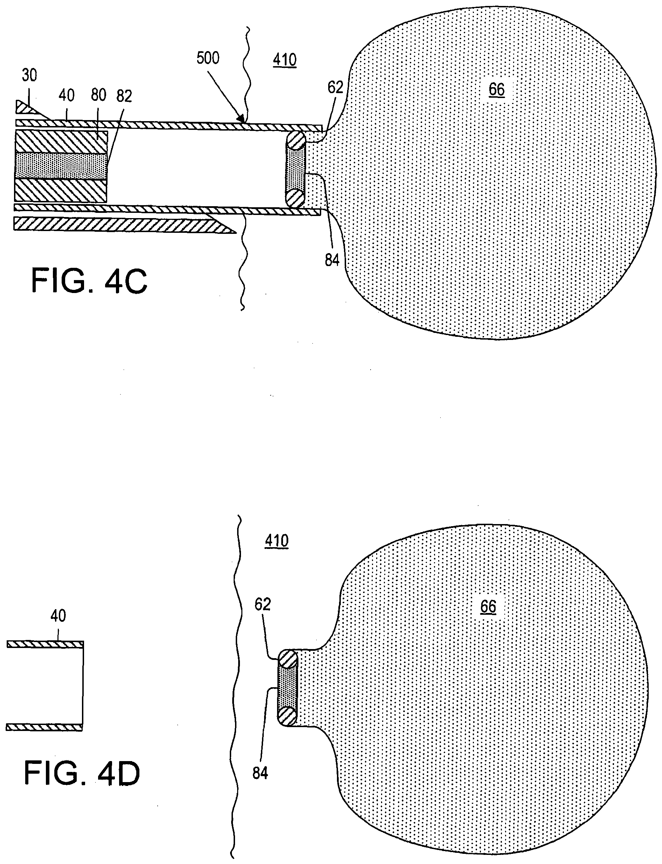

If one desires to seal the capsule 66, an adhesive sealing technique as operationally shown in Figures 4A to 4D may be used. As shown in Figure 4A, an adhesive applicator tube 80 is advanced through the lumen of the membrane introducer tube 40 until the distal tip of the applicator tube 80 engages the seal 62. As shown in Figure 4B, a flowable adhesive 82 is injected into the neck of the capsule 66 so as to fill the neck of the capsule 66. The adhesive 82 is selected to have good adhesion with the seal 62, and may also have good adhesion to the surface of the biomatehal exposed within the neck of the capsule 66. When the applicator tube 80 is withdrawn as shown in Figure 4C, a plug 84 of the adhesive 82 remains in the neck of the capsule 66 proximate the seal 62. The plug 84 completes the seal of the neck of the capsule 66 to prevent contact between the tissue 410 and the biomatehal within the capsule 66. When the capsule 66 as sealed by the seal 62 and the plug 84 is detached from the membrane introducer tube 40 as described previously, and the membrane introducer tube 40 is withdrawn from the tissue 410 as shown in Figure 3D, the sealed capsule 66 remains in place within the tissue 410.

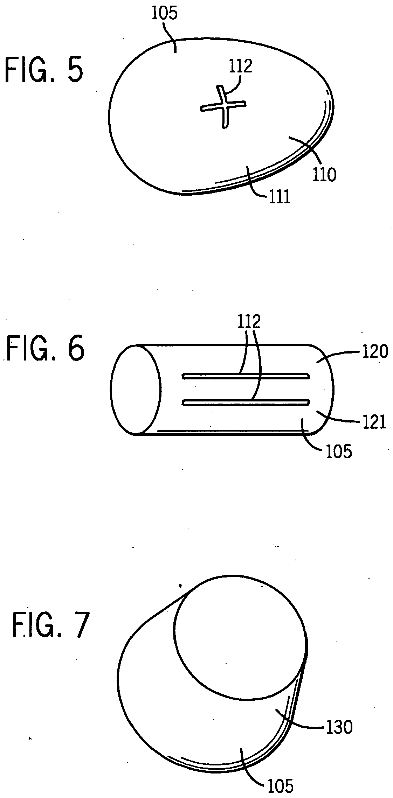

The capsule 66 as illustrated in Figures 3A to 3D and 4A to 4D, is generally spherical in shape. However, other shapes may be used if desired. Figure 5 shows and example of a capsule 110 that has an ovoid shape. Radiopaque markings 112 may be included on the outer surface of the capsule 110 so that the position of the capsule 110 may be located in vivo. Figure 6 shows an example of a capsule 120 that has a cylindrical shape. Radiopaque markings may be included on the outer surface of the capsule 120 to facilitate locating the capsule 120. Figure 7 shows an example of a capsule 130 that has a frustoconical shape.

An illustrative method of operation of the emplacement apparatus 10 is as follows. The distal end of the sheath 200 is inserted into the patient. The needle 30 and the membrane introducer tube 40 are contained within the lumen of the sheath 200 such that the distal end of the emplacement apparatus 10 is generally atraumatic. The sheath distal end 204 is navigated through various bodily passages until generally proximate the injection site 500.

The needle 30 is then deployed with the tip of the needle 30 being inserted into the tissue 410 to a depth that allows for the positioning of the membrane introducer tube 40 within the tissue 410. If not previously in position, the membrane introducer tube 40 with encapsulation membrane 60 in the collapsed position is advanced through the lumen of the needle 30 until positioned at the distal end of the needle 30, at a suitable depth within the tissue 410. Then, the needle 30 is withdrawn from the tissue 410, leaving the encapsulation membrane 60 at the distal end of the membrane introducer tube 40 properly positioned at the desired emplacement site within the tissue 410.

Biomaterial is flowed into the encapsulation membrane 60 to inflate the encapsulation membrane 60 from the collapsed condition into an inflated condition at the emplacement site. Optionally, the neck of the encapsulation membrane 60 is sealed to form the capsule 66. The membrane introducer tube 40 is disengaged from the encapsulation membrane 60 and withdrawn from the tissue 410, leaving the capsule 66 in position at the emplacement site.

According to embodiments, the neck of the encapsulation membrane 60 may be entirely within the tissue at the emplacement location or may extend so as to be accessible outside the tissue at the emplacement location. Corresponding devices and methods for accessing the neck of the encapsulation membrane 60 may be included for either configuration.

Various controls may be disposed about the handle 210 and/or other portions of the emplacement apparatus 10 to aid the physician in directing the distal end of the sheath 200 to the injection site 500, to deploy and retract the needle 30, to position the membrane introducer tube 40 within the lumen of the needle 30, to flow biomaterial into the encapsulation membrane 60, to seal the neck of the capsule 66, to disengage the introducer tube 40 from the capsule 66, and to withdraw the membrane introducer tube 40 from the tissue 410. Suitable controls would be known to one of ordinary skill in the art upon study of this disclosure.

According to embodiments, a kit of parts is disclosed. One or more kits of parts can be envisioned by the person skilled in the art, the kits of parts to perform at least one of the methods herein disclosed. Likewise, directions for use ("DFU") are included and the device may be part of a surgical tray or other packaged accessory set for surgeries. The kit may be a sub-component of a surgical tray.

While embodimentsbeen described in terms of what are presently considered to be the most practical and preferred embodiments, it is to be understood that the disclosure need not be limited to the disclosed embodiments. It is intended to cover various modifications and similar arrangements included within the scope of the claims. The present disclosure includes any and all embodiments of the following claims.

## Claims

1. A capsule emplacement apparatus comprising:
an elongated sheath (200), a needle (30) being movably disposed within a lumen of the sheath (203);
the needle (30) having a tip (34) at a distal (10) end thereof and defining a lumen; the needle (30) disposed within the elongate sheath (200) lumen and configured to translate relative to the elongate sheath (200);
a membrane introducer tube (40) movably disposed within the lumen of the needle (30), the membrane introducer tube (40) defining a lumen within and having a distal end;
a detachable encapsulation membrane (60) defining an interior and an inlet region configured to receive fluid into the interior through the inlet region, the detachable encapsulation membrane (60) being coupled to the distal end of the membrane introducer tube (40) near the inlet region, and being expandable from a collapsed condition, when the detachable encapsulation membrane (60) is disposed within the needle lumen, to an expanded condition, when the detachable encapsulation membrane (60) is extended beyond the needle tip;
a filler tube (64) having a distal end and defining a lumen within, wherein the filler tube (64) when in communication with the inlet region of the detachable encapsulation membrane (60), introduces a fluid into the interior of the detachable encapsulation membrane (60), thereby expanding the detachable encapsulation membrane (60); and
an elongated sealing device disposed within and configured to translated relative to the membrane introducer tube (40), wherein the elongated sealing device configured to seal the detachable encapsulation membrane (60) when positioned at the inlet region of the detachable encapsulation membrane (60).

2. The capsule emplacement apparatus of claim 1, wherein the interior of the encapsulation membrane (60) is in fluid communication through the inlet region with the lumen of the membrane introducer tube (40) for receiving fluid.

3. The capsule emplacement apparatus of claim 1, wherein the elongated sealing device further comprises at least one of an ultrasonic welder, a radio frequency welder, and a thermal welder.

4. The capsule emplacement apparatus of claim 1, wherein the elongated sealing device further comprises an elongated adhesive applicator, further comprising an applicator tube (80) positionable proximate the inlet of the encapsulation membrane (60) for delivering adhesive (82) into the inlet of the encapsulation membrane (60).

5. The capsule emplacement apparatus of claim 1, further comprising a clamping device configured to seal the encapsulation membrane (60) proximate the inlet.

6. The capsule emplacement apparatus of claim 1, further comprising a twisting device configured to seal the encapsulation membrane (60) proximate the inlet.

## Patentansprüche

1. Kapselplatzierungsvorrichtung, die Folgendes umfasst:
eine längliche Hülse (200), wobei eine Nadel (30) beweglich innerhalb eines Lumens (203) der Hülse angeordnet ist,
wobei die Nadel (30) eine Spitze (34) an einem distalen Ende (10) derselben hat und ein Lumen definiert, wobei die Nadel (30) innerhalb des Lumens der länglichen Hülse (200) angeordnet und dafür konfiguriert ist, sich im Verhältnis zu der länglichen Hülse (200) zu verschieben,
eine Membraneinführungsröhre (40), die beweglich innerhalb des Lumens der Nadel (30) angeordnet ist, wobei die Membraneinführungsröhre (40) ein Lumen darin definiert und ein distales Ende hat,
eine lösbare Einkapselungsmembran (60), die ein Inneres und einen Einlassbereich, der dafür konfiguriert ist, durch den Einlassbereich Fluid in das Innere aufzunehmen, definiert, wobei die lösbare Einkapselungsmembran (60) nahe dem Einlassbereich an das distale Ende der Membraneinführungsröhre (40) gekoppelt ist und erweiterbar ist von einem zusammengelegten Zustand, wenn die lösbare Einkapselungsmembran (60) innerhalb des Nadellumens angeordnet ist, zu einem erweiterten Zustand, wenn die lösbare Einkapselungsmembran (60) über die Nadelspitze hinaus ausgedehnt ist,
eine Füllröhre (64), die ein distales Ende hat und ein Lumen darin definiert wobei die Füllröhre (64), wenn sie mit dem Einlassbereich der lösbaren Einkapselungsmembran (60) in Verbindung steht, ein Fluid in das Innere der lösbaren Einkapselungsmembran (60) einleitet, wodurch die lösbare Einkapselungsmembran (60) erweitert wird, und
eine längliche Abdichtungseinrichtung, die innerhalb der Membraneinführungsröhre (40) angeordnet und dafür konfiguriert ist, im Verhältnis zu derselben verschoben zu werden, wobei die längliche Abdichtungseinrichtung dafür konfiguriert ist, die lösbare Einkapselungsmembran (60) abzudichten, wenn sie an dem Einlassbereich der lösbaren Einkapselungsmembran (60) angeordnet ist.

2. Kapselplatzierungsvorrichtung nach Anspruch 1, wobei das Innere der lösbaren Einkapselungsmembran (60) durch den Einlassbereich mit dem Lumen der Membraneinführungsröhre (40) in Fluidverbindung steht.

3. Kapselplatzierungsvorrichtung nach Anspruch 1, wobei die längliche Abdichtungseinrichtung ferner wenigstens eines von einer Ultraschall-Schweißvorrichtung, einer Hochfrequenz-Schweißvorrichtung und einer thermischen Schweißvorrichtung umfasst.

4. Kapselplatzierungsvorrichtung nach Anspruch 1, wobei die längliche Abdichtungseinrichtung ferner einen länglichen Klebstoffapplikator umfasst, wobei sie ferner eine Applikatorröhre (80) umfasst, die nahe dem Einlass der Einkapselungsmembran (60) angeordnet werden kann, um Klebstoff (82) in den Einlass der Einkapselungsmembran (60) abzugeben.

5. Kapselplatzierungsvorrichtung nach Anspruch 1, die ferner eine Klemmeinrichtung umfasst, die dafür konfiguriert ist, die Einkapselungsmembran (60) nahe dem Einlass abzudichten.

6. Kapselplatzierungsvorrichtung nach Anspruch 1, die ferner eine Verdreheinrichtung umfasst, die dafür konfiguriert ist, die Einkapselungsmembran (60) nahe dem Einlass abzudichten.

## Revendications

1. Appareil de mise en place de capsule comprenant :
une gaine allongée (200) et une aiguille (30) disposée de façon mobile à l'intérieur d'une lumière de la gaine (203),
ladite aiguille (30) possédant une pointe (34) à une extrémité distale (10) de celle-ci et définissant une lumière, et ladite aiguille (30) étant disposée à l'intérieur de la lumière de la gaine allongée (200) et étant conçue pour effectuer un mouvement de translation par rapport à la gaine allongée (200),
un tube introducteur de membrane (40) disposé de façon mobile à l'intérieur de la lumière de l'aiguille (30), le tube introducteur de membrane (40) définissant une lumière à l'intérieur et présentant une extrémité distale,
une membrane d'encapsulation amovible (60) définissant un intérieur et une région d'entrée conçue pour recevoir du fluide dans l'intérieur via la région d'entrée, la membrane d'encapsulation amovible (60) étant reliée à l'extrémité distale du tube introducteur de membrane à côté de la région d'entrée, et étant extensible depuis un état écrasé, lorsque la membrane d'encapsulation amovible (60) est disposée à l'intérieur de la lumière de l'aiguille, jusqu'à un état dilaté lorsque la membrane d'encapsulation amovible (60) est déployée au-delà de la pointe d'aiguille,
un tube de remplissage (64) présentant une extrémité distale et définissant une lumière à l'intérieur, lequel tube de remplissage (64), lorsqu'il est en communication avec la région d'entrée de la membrane d'encapsulation amovible (60), introduit un fluide à l'intérieur de la membrane d'encapsulation amovible (60), en dilatant par ce moyen la membrane d'encapsulation amovible (60), et
un dispositif d'obturation allongé disposé à l'intérieur de et conçu pour effectuer un mouvement de translation par rapport au tube introducteur de membrane (40), le dispositif d'obturation allongé étant conçu pour obturer la membrane d'encapsulation amovible (60) lorsqu'il est positionné au niveau de la région d'entrée de la membrane d'encapsulation amovible (60).

2. Appareil de mise en place de capsule selon la revendication 1, dans lequel l'intérieur de la membrane d'encapsulation (60) est en communication fluidique via la région d'entrée avec la lumière du tube introducteur de membrane (40) pour recevoir le fluide.

3. Appareil de mise en place de capsule selon la revendication 1, dans lequel le dispositif d'obturation allongé comprend en plus au moins un dispositif parmi les suivants : dispositif de soudage par ultrasons, dispositif de soudage par radiofréquences et dispositif de soudage thermique.

4. Appareil de mise en place de capsule selon la revendication 1, dans lequel le dispositif d'obturation allongé comprend en plus un applicateur d'adhésif allongé, et comprenant en plus un tube applicateur (80) positionnable à côté de l'entrée de la membrane d'encapsulation (60) pour distribuer de l'adhésif (82) dans l'entrée de la membrane d'encapsulation (60).

5. Appareil de mise en place de capsule selon la revendication 1, comprenant en plus un dispositif de serrage conçu pour obturer la membrane d'encapsulation (60) à côté de l'entrée.

6. Appareil de mise en place de capsule selon la revendication 1, comprenant en plus un dispositif de torsion conçu pour obturer la membrane d'encapsulation (60) à côté de l'entrée.
